# EUROPEAN PATENT APPLICATION

(11) **EP 3 578 216 A1**
(43) Date of publication of application: **11.12.2019**
(21) Application number: 18176839.1
(22) Date of filing: 08.06.2018
(51) Int. Cl.: A61M 15/00

(54) **INHALER**

(71) Applicant: Presspart Manufacturing S.A., 43720 L'Arboc del Penedes, Tarragona (ES)
(72) Inventor: Torres Muniesa, Victor, 43720 L'Arboç, Tarragona (ES); Herrera Martínez, Paloma, 43720 L'Arboç, Tarragona (ES); Busto Sotil, Santiago, 43720 L'Arboç, Tarragona (ES); Valencia Pellisa, David, 43720 L'Arboç, Tarragona (ES)
(74) Representative: Uexküll & Stolberg

(57) **Abstract**

An inhaler (1) has an hollow inhaler body (2) for retaining an aerosol container with a dispensing valve at a valve end of the container. The inhaler body (2) comprises a first open end (3) sized and arranged to receive the aerosol container with a dispensing valve and a second open end (4) through which a dose of medicament is dispensed as an aerosol. The second open end (4) is sized and arranged to be coupled to the mouth or nasal cavities of a patient. The inhaler body (2) further comprises a nozzle block (5) which extends from a base part (6) located at a base portion (12) of the inhaler body (2) towards the first open end (3) and against which the dispensing valve of the aerosol container is depressed to release a dose of a medicament. The nozzle block (5) has a fluid flow path (7) extending therethrough such that the dose of medicament being released from the valve is passed to the nozzle block (5) and exits the nozzle block (5) as an aerosol in a direction towards the second open end (4) of the inhaler body (2).

The base part (6) is formed as a part separate from the inhaler body (2) and is insertable through an opening (11) in the base portion (12) of the inhaler body (2) together with the nozzle block (5). The base part (6) is configured to close the opening (11) of the base portion (12) upon insertion into the inhaler body (2).

## Description

### TECHNICAL FIELD

The present invention relates to an inhaler having a hollow inhaler body for retaining an aerosol container with a dispensing valve at a valve end of the container. The inhaler body comprises a first open end sized and arranged to receive the aerosol container with a dispensing valve and a second open end through which a dose of medicament is dispensed as an aerosol. The second open end is sized and arranged to be coupled to the mouth or nasal cavities of a patient. The inhaler body further comprises a nozzle block which extends from a base part located at a base portion of the inhaler body towards the first open end and against which the dispensing valve of the aerosol container is depressed to release a dose of a medicament. The nozzle block has a fluid flow path extending therethrough such that the dose of medicament being released from the valve is passed to the nozzle block and exits the nozzle block as an aerosol in a direction towards the second open end of the inhaler body.

### BACKGROUND OF THE INVENTION

Inhalers, in particular metered dose inhalers (MDIs), are medication delivery devices which deliver a pharmaceutical formulation including one or more pharmaceutical active compounds to a human or another mammalian patient. Typically, the pharmaceutical formulation is delivered by the MDIs in unit doses in the form of an aerosol. Each actuation of the MDIs delivers one unit dose. The unit dose is expelled by the MDIs and is taken into the body of the patient on inhalation, via the nose or mouth. The pharmaceutical formulation is delivered to or via the respiratory tract, notably to the lungs, of the patient on inhalation. Metered dose inhalers are typically used for the treatment of respiratory infections and disorders including respiratory tract infections, obstructive lung disease, inflammatory lung disease and chronic obstructive pulmonary disease. Asthma treatment is a particularly common use of MDIs

ES 1069857 U discloses such a metered dose inhaler having an angular body with a notably cylindrical vertical part and a lower part. The lower part protrudes forward, constituting an outlet opening forming an angle of greater than 90° with the vertical part. An outlet structure is positioned on the lower base of the vertical part and comprises several holes in order to direct aerosol from a container being received in the vertical part to the outlet opening.

Typically, inhalers are manufactured according to customer specifications. After delivery to the customer the inhalers are then equipped with an aerosol container and brought to the market. Specifications of different customers often only vary with regard to the design of the nozzle block. This is because the design of the nozzle block significantly influences the spray pattern of the aerosol exiting the nozzle towards the second open end of the inhaler body. Moreover, the nozzle block design depends amongst others on the formulation of the medicament to be used in combination with the inhaler. However, the whole inhaler has to be designed and manufactured individually in order to address the needs of every customer which is rather time consuming and expensive.

Moreover, inhalers are preferably manufactured under use of the injection molding technique by which melted plastic particles are injected into moulds where they solidify and take the appearance of an inhaler body. As the geometry of an inhaler body comprises several openings and undercuts its geometry is quite complex. Accordingly, the moulds comprise several parts which are movable relative to one another in order to allow removement of the inhaler body from the mould. Consequently tooling, in particular the moulds used for the injection molding, are very expensive.

It is an object of the present invention to provide a cost effective inhaler which allows a simplified production.

### SUMMARY OF THE INVENTION

This object is achieved by an inhaler comprising the features of claim 1, preferred embodiments are set out in the dependent claims.

According to the present invention the base part is formed as a part separate from the inhaler body and is insertable through an opening in the base portion of the inhaler body together with the nozzle block, wherein the base part is configured to close the opening of the base portion upon insertion into the inhaler body.

Forming the base part as well as the nozzle block as a part separate from the inhaler body is a cost effective way to individually design the nozzle block according to customer needs, whereas the design of the inhaler body remains the same. Additionally, the base part as well as the nozzle block may be designed as a part being compatible with a range of inhaler bodies having different sizes and/or different shapes. Moreover, the design of the base part as well as the nozzle block can be optimized with regard to the manufacturing process, in particular with regard to the injection molding of the nozzle block, without having to consider producibility of the base part, the nozzle block and the inhaler body in one single mould. Additionally, separating the manufacturing of the inhaler body and the base part as well as the nozzle block allows cheaper tooling for both the base part together with the nozzle block and the inhaler body. Consequently, the production costs for an inhaler and thus the price of sale decreases.

Preferably, the inhaler according to the present invention may be used as a metered dose inhaler with or without an additional dose counter which may be a mechanical and/or electronic dose counter. Optionally, a mechanical dose counter is located vertically above the nozzle block. Alternatively or additionally an electronic dose counter may be located between a rear side of the inhaler body and the aerosol container or a front side of the inhaler body and the aerosol container. Preferably, the mechanical dose counter is mounted on the base part and is inserted together with the base part and the nozzle block through the opening in the base portion of the inhaler body. Thus, access of a user to the dose counter and thus manipulation of the same is omitted. In case the mechanical dose counter is mounted on the base part, a standard mechanical dose counter as known from the prior art can be used. There are no modifications of the dose counter needed with regard to the fixation of the dose counter relative to the inhaler body. Preferably, the base part comprises the same mounting points for mounting the dose counter to the base part and the inhaler body, respectively, as a one piece inhaler body. Use of a standard dose counter further reduces the production costs.

In an embodiment of the present invention the nozzle block is formed as a part separate from the base part and is mountable thereto, wherein the base part comprises a receiving portion for receiving the nozzle block. This allows an individual design of an inhaler which fits individual needs of the customers while being more flexible regarding the choice of different shapes and sizes of the nozzle block, the base part and the inhaler body compared to an integral design of the nozzle block and the base part. Moreover, forming the nozzle block separate from the base part also reduces costs as the complexity of the tooling is further reduced. Optionally, the nozzle block may be formed as a standard component which may be used in different inhaler bodies with different designs.

In another embodiment of the present invention the receiving portion has an essentially cylindrical shape and comprises opposing protrusions which protrude radially inward. Preferably, the nozzle block comprises recesses which correspond to the protrusions of the receiving portion such that the protrusions of the receiving portion engage with the recesses of the nozzle block upon insertion of the nozzle block into the receiving portion.

Preferably the nozzle block is connected to the base part via a form fitting connection. The form fitting connection may be configured to withstand the forces which occur during operation of the inhaler, in particular during depression and release of the dispensing valve. Preferably, the nozzle block has at least partially a cylindrical shape, wherein the nozzle block comprises a radially outward extending lower edge which engages with a slot in the receiving portion. Upon engagement of the lower edge and the slot, axial movement of the nozzle block towards the first open end is preferably omitted. The form fitting connection allows an easy assembling of the nozzle block and the base part.

In an embodiment of the present invention the base part has a bottom side which is flush with a bottom side of the inhaler body. This arrangement not only enables an appealing appearance of the inhaler but also avoids disturbing edges in transition between the bottom side of the inhaler body and the bottom side of the base part.

In another embodiment the base part comprises a corrugation at its bottom side. The bottom side of the base part is often used by patients to hold the inhaler during inhalation. As the corrugation allows secure gripping of the inhaler during inhalation an accidently dropping of the inhaler is avoided.

Preferably, the base part is connected to the base portion of the inhaler body via a form fitting connection. The form fitting connection may be configured to withstand the forces during actuation of the aerosol canister, i.e. the pressing of the aerosol container towards the bottom side of the inhaler body in order to release a dose of medicament. Moreover, the form fitting connecting preferably secures proper positioning of the nozzle block with regard to the second open end of the inhaler body. In particular the form fitting connection secures positioning of the nozzle block in the inhaler body such that the dose of medicament being released from the valve exits the nozzle block as an aerosol in a direction towards the second open end of the inhaler body. Additionally, the form fitting connection preferably allows an airtight connection between the base part cover and the base portion of the inhaler body such that during inhalation no air uncontrollably exits or enters the inhaler body.

In an embodiment of the present invention the form fitting connection is a snap fit connection. Snap fit connections allow easy and fast assembling of the base part and the nozzle block and the inhaler body. Preferably, the base part comprises a ledge on a first side which engages with the inhaler body in proximity to its second open end, whereas the base part further comprises a snap-in tongue on a second side which is clipped into a corresponding cut out in the base portion of the inhaler body. Preferably, the second side opposes the first side. More preferably, the first side is a front side of the base part and the second side is a back side of the base part.

In another embodiment the nozzle block is formed of transparent material. This allows visual inspection of the nozzle block after fabrication. In particular, this allows the measurement of the nozzle block by the use of vision systems which don't require a specific handling or physical change (e.g. cutting, adding liquids for increasing contrast etc.). This enables a cheap, fast and 100% inline inspection check for nozzle blocks and thus total batch traceability. Vision systems typically allow to capture a digital image of a part which is to be measured. The measurements are then taken from the image manually or automatically in a subsequent step. Preferably the vision system comprises a camera or any other device which is capable of taking an image of the part to be measured.

Optionally, the fluid flow path of the nozzle block is formed by a sump configured to receive the dispensing valve of the aerosol container, a nozzle configured to aerosolize the dose of medicament being released from the dispensing valve and a channel extending from the sump to the nozzle. In order to ensure a proper functioning of the nozzle block and that the molded nozzle block is dimensionally within the specification, it is, among other dimensions, required to measure the diameter at the transition of the channel to the nozzle (orifice diameter) as well as the length of the channel (jet length).

In an embodiment of the present invention the nozzle block has an upper portion, and a lower portion positioned adjacent to one another, wherein the lower portion comprises opposing plane side surfaces which are arranged parallel to one another. The plane side surfaces allow recording of a non-distorted image of the interior of the nozzle block as the rays, in particular light rays, enter and exit the nozzle block preferably at a right angle with regard to the plane side surfaces such that the rays are not deflected. This enables true measurements of the interior of the nozzle block.

In another embodiment side surfaces of the lower portion are arranged parallel to a fist axis defined by the channel connecting the sump and the nozzle of the nozzle block and are arranged parallel to a second axis defined by the nozzle block extending from the base part towards the first open end of the inhaler body. Such an arrangement of the side surfaces allows direct measuring of the jet length as well as the orifice in between the channel and the nozzle.

In a preferred embodiment the upper part of the nozzle block has a cylindrical shape and comprises opposing recesses, which each have a plane base surface, wherein the base surfaces are arranged parallel to one another. Optionally the recesses correspond to the radially inward extending protrusions of the receiving portion of the base part.

Preferably, the protrusions of the receiving portion form a stop for the recesses and thus keep the nozzle block in an upright position in the receiving portion such that the nozzle block points towards the first open end of the inhaler body. Due to the upright position the lower edge of the nozzle block is kept in engagement with the slot of the receiving portion. Thus, the engagement of the recesses and the protrusions additionally prevents movement of the nozzle block along the longitudinal axis defined by the nozzle block extending from the base part towards the first open end of the inhaler body.

Preferably, the nozzle block has a supporting leg for vertically supporting the nozzle block in the receiving portion, wherein the supporting leg comprises a T-shaped cross sectional area having a stem and two opposing arms, wherein the stem forms a rib which extends from the upper portion along the lower portion and, wherein the rib is adapted to engage with a vertically extending groove in the receiving portion. The T-shaped cross sectional area extends lateral to an axis defined by the nozzle block extending from the base part towards the first open end of the inhaler body. Upon engagement of the rib and the vertically extending groove in the receiving portion the orientation of the nozzle block around the longitudinal axis defined by the nozzle block extending from the base part towards the first open end of the inhaler body is set. This ensures that a dose of a medicament being released from the valve of the aerosol container exits the nozzle block as an aerosol in a direction towards the second open end of the inhaler body. The rib of the nozzle block and the groove in the receiving portion are engaged upon insertion of the nozzle block into the receiving portion.

In another embodiment the inhaler comprises an insert configured to be inserted into the inhaler body through its first open end, wherein the insert has ribs which extend from the first open end towards the base portion of the inhaler body and which are adapted to abut the aerosol container upon insertion into the inhaler body. Preferably, the insert is available in different sizes which differ in the size of the ribs in between the inhaler body and the aerosol container and thus in a radial direction, namely in a direction lateral to the second axis. The insert allows the adaption of the inhaler body to receive aerosol containers of different sizes, in particular of different diameters. Thus, by use of inserts having different sizes one single inhaler body may be adapted to accommodate aerosol containers of different sizes, thereby reducing costs of tooling and production costs.

The invention will now be described in connection with one exemplary embodiment shown in the figures in which:
- Figure 1: shows a perspective view of an inhaler according to the present invention,
- Figure 2: shows a cross-sectional view of the inhaler of Figure 1,
- Figure 3: shows an exploded view of a base part together with a nozzle block and a dose counter,
- Figure 4: shows a perspective view of the nozzle block of Figure 3,
- Figure 5: shows a perspective rear view of the nozzle block of Figure 3,
- Figure 6A: shows a sectional top view along the line A-A in Figure 2,
- Figure 6B: shows a detail Y of Figure 6A,
- Figure 7A: shows a sectional top view along the line B-B in Figure 2 and
- Figure 7B: shows a detail Z of Figure 7A.

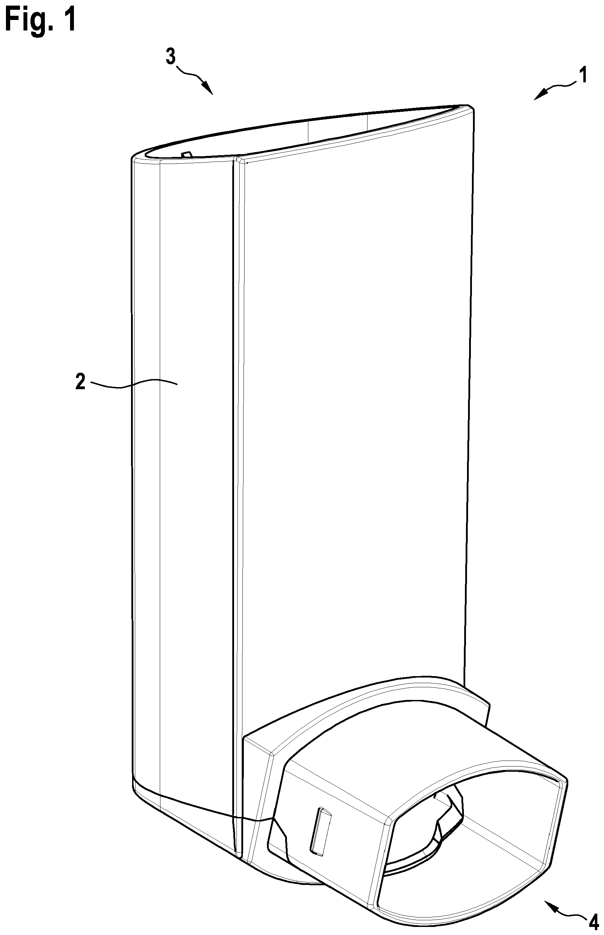

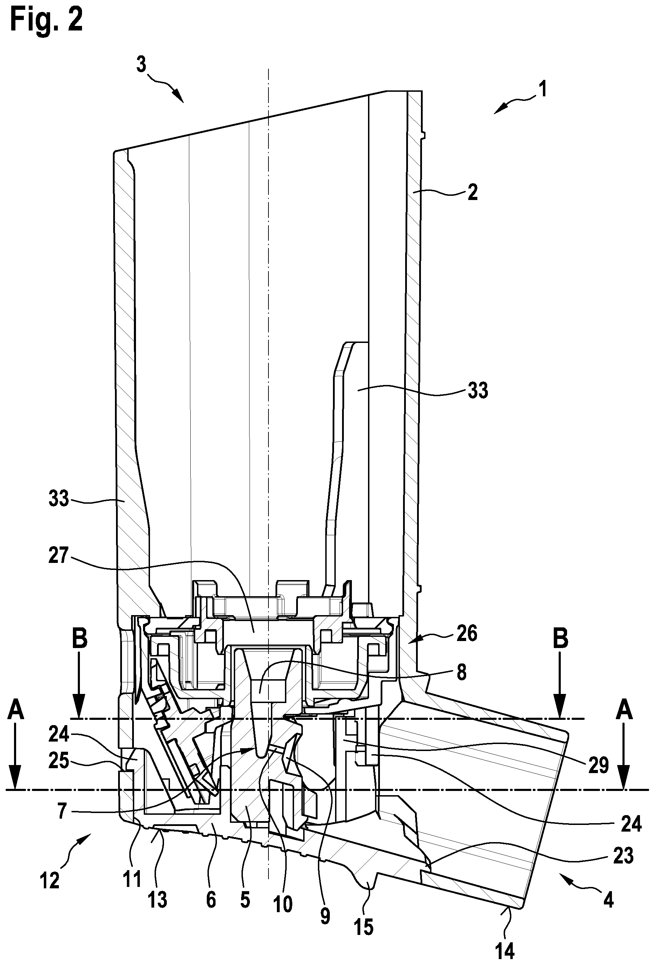

Figures 1 and 2 show an inhaler 1 with a hollow inhaler body 2 for retaining an aerosol container (not shown) with a dispensing valve at a valve end of the container. The inhaler 1 comprises a first open end 3 sized and arranged to receive the aerosol container with a dispensing valve and a second open end 4 through which a dose of a medicament is dispensed as an aerosol. The second open end 4 is sized and arranged to be coupled to the mouth or nasal cavities of a patient (not shown). The inhaler body 2 forms an angular hollow tubular body extending from the first open end 3 towards the second open end 4.

The inhaler body 2 further comprises ribs 33 which extend from the first open end 3 towards a base portion 12 of the inhaler body 2. The ribs 33 are adapted to abut the aerosol container upon its insertion into the inhaler body 2. Preferably, the ribs 33 are arranged on an insert (not shown) configured to be inserted into the inhaler body 2 through its first open end 3. The insert may be fixed to the inhaler body 2. Optionally, the insert may be exchangeable such that multiple inserts with different sizes of the ribs 33 may alternatively be inserted into the inhaler body 2 in order to accommodate aerosol containers of different sizes.

The inhaler body 2 further comprises a nozzle block 5 which extends from a base part 6 located at the base portion 12 of the inhaler body 2 towards the first open end 3 and against which the dispensing valve of the aerosol container is depressed to release a dose of medicament. The nozzle block 5 has a fluid flow path 7 extending therethrough such that the dose of medicament being released from the valve is passed to the nozzle block 5 and exits the nozzle block 5 as an aerosol in a direction towards the second open end 4 of the inhaler body 2.

The fluid flow path 7 of the nozzle block 5 is formed by a sump 8 configured to receive the dispensing valve of the aerosol container, a nozzle 9 configured to aerosolize the medicament being released from the dispensing valve and a channel 10 extending from the sump 8 to the nozzle 9.

The base part 6 is formed as a part separate from the inhaler body 2 and is insertable through an opening 11 in the base portion 12 of the inhaler body 2 together with the nozzle block 5. The base part 6 is configured to close the opening 11 of the base portion 12 upon insertion into the inhaler body 2.

The base part 6 has a bottom side 13 which is flush with a bottom side 14 of the inhaler body 2. The base part 6 comprises a corrugation 15 at its bottom side 13 for allowing proper handling of the inhaler 1 during inhalation.

The base part 6 is connected to the base portion 12 of the inhaler body 2 via a form fitting connection such as a snap-fit connection. Accordingly, the base part 6 comprises a ledge 23 on a front side which engages with the inhaler body 2 in proximity to its second open end 4. The base part 6 further has a snap-in tongue 24 at its back side which is clipped into a corresponding cut out 25 in the base portion 12 of the inhaler body 2. Optionally, the nozzle block 5 is formed of transparent material, in particular transparent plastic material.

The inhaler 1 further comprises a mechanical dose counter 26 which is located vertically above the nozzle block 5. The mechanical dose 26 counter comprises a through hole 27 through which the nozzle block 5 extends at least partially and through which the dispensing vale (not shown) is inserted in order to be received by the sump 8 of the nozzle block 5. The mechanical dose counter 26 comprises snap-in tongues 24 which engage with corresponding projections 29 at the base part 6 to form a form fitting connection.

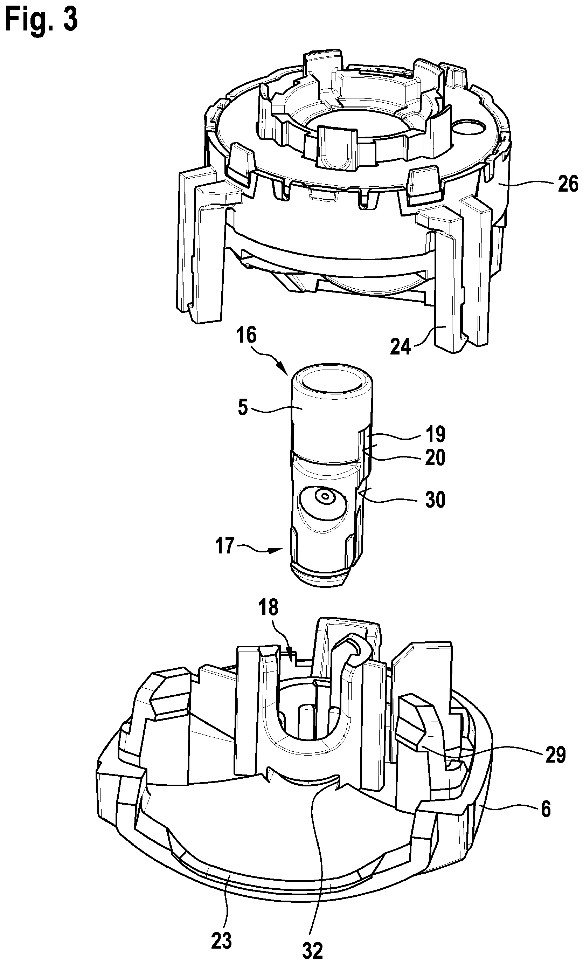

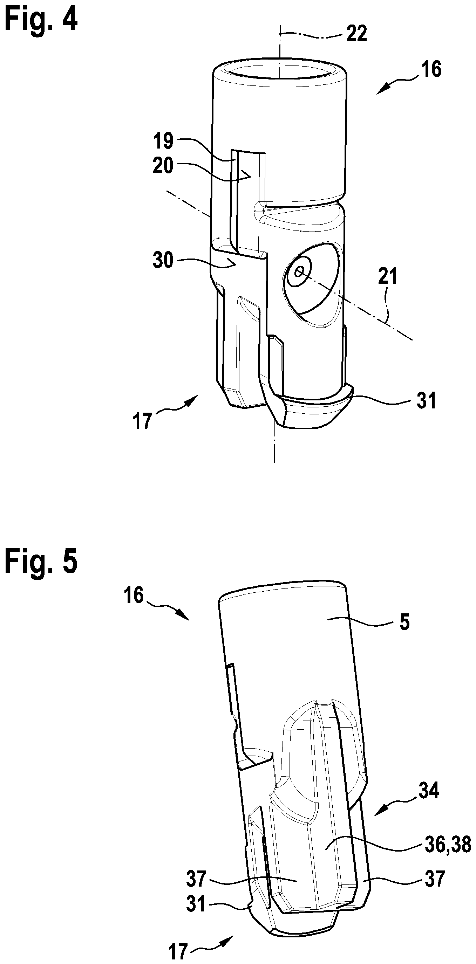

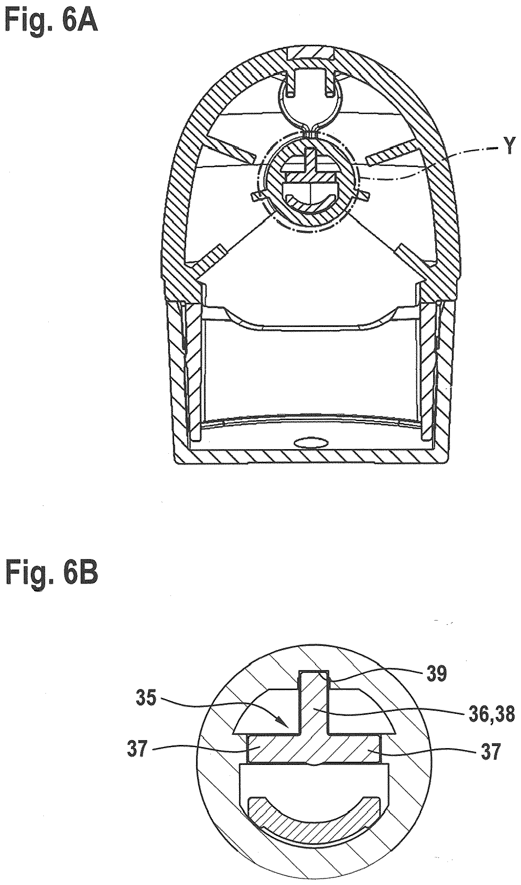

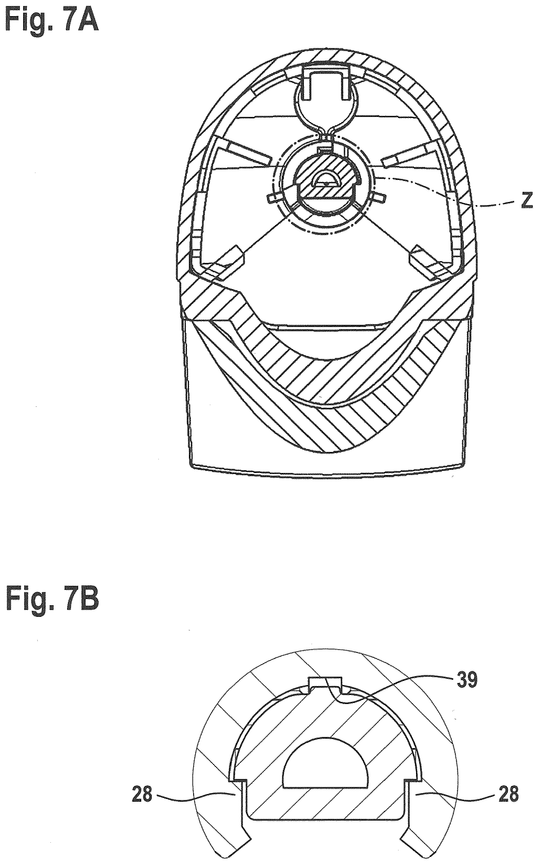

Figures 3 to 7B show the nozzle block 5 and its position in the base part 6 in more detail.

The nozzle block 5 is formed as a part separate from the base part 6 and is mountable thereto. The base part 6 comprises a receiving portion 18 for receiving the nozzle block 5. The receiving portion 18 has an essentially cylindrical shape and comprises opposing protrusions 28 which radially protrude inward.

The nozzle block 5 has an upper portion 16, and a lower portion 17 positioned adjacent to one another. The upper portion 16 of the nozzle block 5 has a cylindrical shape and comprises opposing recesses 19, which each have a plane base surface 20, wherein the base surfaces 20 are arranged parallel to one another. The opposing recesses 19 are configured to engage with the opposing protrusions 28 of the receiving portion 18 of the base part 6 upon insertion of the nozzle block 5 into the receiving portion 18.

The lower portion 17 of the nozzle block 5 comprises opposing plane side surfaces 30 which are arranged parallel to one another. The side surfaces 30 of the lower portion 17 are arranged parallel to a fist axis 21 defined by the channel 10 connecting the sump 8 and the nozzle 9 of the nozzle block 5 (Fig. 2) and are arranged parallel to a second axis 22 defined by the nozzle block 5 extending from the base part 6 towards the first open end 3 of the inhaler body 2.

The nozzle block 5 has a supporting leg 34 for vertically supporting the nozzle block 5 in the receiving portion 18. The supporting leg 34 comprises a T-shaped cross sectional area 35 having a stem 36 and two opposing arms 37. The stem 36 forms a rib 38 which extends from the upper portion 16 along the lower portion 17 and is adapted to engage with a vertically extending groove 39 in the receiving portion 18.

The nozzle block 5 further comprises at its lower portion 17 a radially outward extending lower edge 31 which engages with a slot 32 in the receiving portion 18. The lower edge 31 of the nozzle block 5 together with the slot 32 of the receiving portion 18 form a form fitting connection of the nozzle block 5 and the base part 6.

The assembly of the inhaler 1 is explained in the following in detail with respect to figures 1 to 7B:
In a first step the nozzle block 5 is partly inserted into the receiving portion 18 of the base part 6, such that the rib 38 engages with the vertically extending groove 39 in the receiving portion 18. Upon engagement of the rib 38 and the groove 39 the orientation of the nozzle block 5 around the second axis 22 relative to the base part 6 is set.

Upon further movement of the nozzle block 5 towards the base part 6 and into the receiving portion 18 the recesses 19 of the upper portion 16 of the nozzle block 5 engage with the radially inward extending protrusions 28 of the receiving portion 18, forcing the nozzle block 5 in an upright position.

The nozzle block 5 is inserted into the receiving portion 18 until the radially outward extending edge 31 at the lower portion 17 of the nozzle block 5 engages with the corresponding slot 32 in the base part 6. Upon engagement of the lower edge 31 of the nozzle block 5 and the slot 32 of the base part 6 the axial position of the nozzle block 5 along the second axis 22 relative to the base part 6 is fixed.

In a second step the mechanical dose counter 26 is positioned on top of the base part 6 such that the nozzle block 5 at least partially protrudes through the through hole 27 of the dose counter 26. The snap-in tongues 24 of the mechanical dose counter 26 engage with the projections 29 of the base part 6 to form a form fitting connection between the mechanical dose counter 26 and the base part 6.

In a third step the base part 6 together with the nozzle block 5 and the mechanical dose counter 26 is inserted into the inhaler body 2 through the opening 11 in the base portion 12 of the inhaler body 2. The mechanical dose counter 26 rests in a vertical direction against ribs 33 (Fig. 2) which are positioned inside the inhaler body 2. The ledge 23 on the front side of the base part 6 is engaged with the inhaler body 2, whereas the snap-in tongue 24 at the back side of the base part 6 is clipped into the corresponding cut out 25 in the base portion 12 of the inhaler body 2.

### Reference numerals

- 1: inhaler
- 2: inhaler body
- 3: first open end (inhaler body)
- 4: second open end (inhaler body)
- 5: nozzle block
- 6: base part
- 7: fluid flow path (nozzle block)
- 8: sump (nozzle block)
- 9: nozzle (nozzle block)
- 10: channel (nozzle block)
- 11: opening (base portion)
- 12: base portion (inhaler body)
- 13: bottom side (nozzle block cover)
- 14: bottom side (inhaler body)
- 15: corrugation (cover)
- 16: upper portion (nozzle block)
- 17: lower portion (nozzle block)
- 18: receiving portion (base part)
- 19: recesses (nozzle block)
- 20: base surface (recesses)
- 21: first axis (channel)
- 22: second axis (nozzle block)
- 23: ledge (base part)
- 24: snap in tongue (base part/dose counter)
- 25: cut out (inhaler body)
- 26: dose counter
- 27: through hole (dose counter)
- 28: protrusions (base part)
- 29: projection (receiving portion)
- 30: side surface (nozzle block)
- 31: lower edge (nozzle block)
- 32: slot (base portion)
- 33: ribs (inhaler body)
- 34: supporting leg (nozzle block)
- 35: cross sectional area (supporting leg)
- 36: stem (supporting leg)
- 37: arms (supporting leg)
- 38: rib (supporting leg)
- 39: groove (receiving portion)

## Claims

1. An inhaler having a hollow inhaler body (2) for retaining an aerosol container with a dispensing valve at a valve end of the container, the inhaler body (2)comprising:
- a first open end (3) sized and arranged to receive the aerosol container with a dispensing valve;
- a second open end (4) through which a dose of medicament is dispensed as an aerosol, the second open end (4) being sized and arranged to be coupled to the mouth or nasal cavities of a patient;
- a nozzle block (5) which extends from a base part (6) located at a base portion (12) of the inhaler body (2) towards the first open end (3) and against which the dispensing valve of the aerosol container is depressed to release a dose of a medicament, the nozzle block (5) having a fluid flow path (7) extending therethrough such that the dose of medicament being released from the valve is passed to the nozzle block (5) and exits the nozzle block (5) as an aerosol in a direction towards the second open end (4) of the inhaler body (2);
wherein
the base part (6) is formed as a part separate from the inhaler body (2) and is insertable through an opening (11) in the base portion (12) of the inhaler body (2) together with the nozzle block (5), wherein the base part (6) is configured to close the opening (11) of the base portion (12) upon insertion into the inhaler body (2).

2. Inhaler according to claim 1, wherein the nozzle block (5) is formed as a part separate from the base part (6) and is mountable thereto, wherein the base part (6) comprises a receiving portion (18) for receiving the nozzle block (5).

3. Inhaler according to claim 2, **characterized in that** the receiving portion (18) has an essentially cylindrical shape and comprises opposing protrusions (28) which protrude radially inward.

4. Inhaler according to claim 2, **characterized in that** the nozzle block (5) is connected to the base part (6) via a form fitting connection.

5. Inhaler according to any of the preceding claims, **characterized in that** the base part (6) has a bottom side (13) which is flush with a bottom side (14) of the inhaler body (2) .

6. Inhaler according to claim 4, wherein the base part (6) comprises a corrugation (15) at its bottom side (13).

7. Inhaler according to any of the preceding claims, **characterized in that** the base part (6) is connected to the base portion (12) of the inhaler body (2) via a form fitting connection.

8. Inhaler according to claim 7, **characterized in that** the base part (6) comprises a ledge (23) on a first side which is configures to engage with the inhaler body (2) in proximity to its second open end (4) and that the base part (6) further has a snap-in tongue (24) at a opposing second side which is configured to clip into a corresponding cut out (25) in the base portion (12) of the inhaler body (2) to establish the form fitting connection between the base part (6) and base portion (12) of the inhaler body (2).

9. Inhaler according to any of the preceding claims, **characterized in that** the nozzle block (5) is formed of transparent material.

10. Inhaler according to any of the preceding claims, **characterized in that** the fluid flow path (7) of the nozzle block (5) is formed by a sump (8) configured to receive the dispensing valve of the aerosol container, a nozzle (9) configured to aerosolize the dose of medicament being released from the dispensing valve and a channel (10) extending from the sump (8) to the nozzle (9).

11. Inhaler according to any of the preceding claims, **characterized in that** the nozzle block (5) has an upper portion (16), and a lower portion (17) positioned adjacent to one another, wherein the lower portion (17) comprises opposing plane side surfaces (30) which are arranged parallel to one another.

12. Inhaler according to claims 10 and 11, **characterized in that** the side surfaces (30) of the lower portion (17) are arranged parallel to a fist axis (21) defined by the channel (10) connecting the sump (8) and the nozzle (9) of the nozzle block (5) and are arranged parallel to a second axis (22) defined by the nozzle block (5) extending from the base part (6) towards the first open end (3) of the inhaler body (2).

13. Inhaler according to claim 11, **characterized in that** the upper portion (16) of the nozzle block (5) has a cylindrical shape and comprises opposing recesses (19), which each have a plane base surface (20), wherein the base surfaces (20) are arranged parallel to one another.

14. Inhaler according to claim 11, **characterized in that** the nozzle block (5) has a supporting leg (34) for vertically supporting the nozzle block (5) in the receiving portion (18), wherein the supporting leg (34) comprises a T-shaped cross sectional area (35) having a stem (36) and two opposing arms (37), wherein the stem (36) forms a rib (38) which extends from the upper portion (16) along the lower portion (17) and, wherein the rib (38) is adapted to engage with a vertically extending groove (39) in the receiving portion (18) .

15. Inhaler according to any of the preceding claims, **characterized in that** the inhaler (1) comprises an insert configured to be inserted into the inhaler body (2) through its first open end (3), wherein the insert has ribs (33) which extend from the first open end (3) towards the base portion (12) of the inhaler body (2) and which are adapted to abut the aerosol container upon insertion into the inhaler body (2) .
